# EUROPEAN PATENT APPLICATION

(11) **EP 1 811 071 A1**
(43) Date of publication of application: **25.07.2007**
(21) Application number: 06000989.1
(22) Date of filing: 18.01.2006
(51) Int. Cl.: D04H 1/64, A61F 13/15

(54) **Latex bonded airlaid fabric and its use**

(71) Applicant: Celanese Emulsions GmbH, 61476 Kronberg/Ts. (DE)
(72) Inventor: Scott, Paul, Surrey KT17 2LQ (GB)
(74) Representative: Ackermann, Joachim

(57) **Abstract**

Disclosed is a nonwoven airlaid fabric comprising a combination of synthetic fibres and of natural fibres which has been consolidated by application and crosslinking of a crosslinkable polymer dispersion.

These products can be used as acquisition and distribution layers in personal care products.

## Description

### FIELD OF THE INVENTION

The present invention relates to nonwoven fabrics which can be used as a component in the manufacture of absorbent articles such as disposable diapers, sanitary napkins and panty liners. More specifically, the present invention relates to acquisition distribution layers (hereinafter "ADL") and materials for such ADL's, which are very suitable to be used with thin products potentially containg a relatively high concentration of super-absorbent polymer material.

### BACKGROUND OF THE INVENTION

Disposable absorbent articles are broadly available and consumers are used to a high performance for the collecting and retaining of body fluids. However, besides this consumers do expect an improved wearing comfort of such articles, and namely an improved dryness of those articles.

Typically, such articles comprise multiple absorbent members, at least one member being primarily designed to store liquid, and at least one other member primarily designed to acquire and / or distribute liquid.

At least the storage member will often comprise super-absorbent material, which is admixed with the traditionally used pulp fiber material. Such super-absorbent materials can absorb many times (e.g. 10 or more times) their own weight. Modem products employ high concentrations of super-absorbent materials, that is concentrations in excess of 50% of the total weight of the storage member. These products achieve a high absorbing capacity with a very thin storage member and are thereby typically overall thin products. While super-absorbent materials can store very large amounts of liquid, they are offen not able to distribute the liquid from the point of impact to more remote areas of the absorbent article and to acquire the liquid as fast as it may be received by the article.

For this reason acquisition distribution layers are used, which provide for the interim acquisition of large amounts of liquid and which often also allow for the distribution of liquid. Thereby the ADL plays a key rote in using the whole absorbent capacity provided by the storage member.

ADL comprising absorbent articles are disclosed, for example in WO-A-00/151651 or in WO-A-2005/016,207.

By definition, an ADL is a sub-layer arranged between a top sheet and an absorbent core whose primary function is to transport fluid insults from the outer surface of a final product, such as a diaper or a feminine hygiene product, to the inner core imparting fast strike through and low rewet. To achieve these properties, the ADL design is typically a bulky, open structured, resilient nonwoven offering good compression recovery regaining its loft quickly after compaction. Such configurations have historically been produced by the drylaid-carded process using SBR (styrenebutadiene-rubber) as the preferred consolidation chemistry with 100 % crimped polyester fibres.

The airlaid manufacturing process is capable of running at faster machine speeds than drylaid carded and offers greater flexibility to the roll goods producer in terms of fibre selection and method of fabric consolidation.

Different ADL types have already been disclosed in the prior art. Typically these webs comprise cellulose fibers which are bonded using a binder, as disclosed in CA-A-2,160,757. The airlaid technology has also been proposed for the manufacture of ADL's, for example in WO-A-20051016,207 and in WO-A-2005/041,815.

JP-A-09178,423 discloses a paper made of nonwoven fabric made by addition of a foamy crosslinkable binder to a nonwoven web, compressing said web with said binder between the nip of two rollers to establish a concentration gradient of said binder in the width direction of said web and heat treating the paper to crosslink said binder.

US-A-5,938,995 discloses a compression resistant cellulosic-based fabric having hith rates of absorbency. The fabric is preferably made by an airlaid process and requires application of stiffened cellulosic fibers which have been treated prior to web formation with a chemical stiffening agent being capable of effecting the crosslinking of said fibers. As a binder a non-crosslinkable or a crosslinkable polymeric binder is used.

US-A-2004/0242106 discloses nonwoven webs bonded together with a selected crosslinkable binder comprising vinyl acetate ethylene units and possessing galss transition temperatures of less than 10°C. This document discloses airlaid webs formed from blends of cellulose and bicomponent synthetic fibers.

US-A-200310089443 discloses a dry-laid web comprising hollow synthetic fibers, absorbent, for example wood fluff pulp, and binder system. Different conventional latex type binders are disclosed.

US-A-2003/0003830 discloses an air-laid web comprising synthetic fibers, absorbent, for example wood fluff pulp, and binder. Different conventional latex type binders are disclosed.

US-A-2004101039T0 discloses a single layer air-laid web comprising hollow synthetic fibers, absorbent, for example wood fluff pulp, and binder. Different conventional latex type binders are disclosed.

DE-A-102 18 259 discloses embossed airlaid products comprising a nonwoven web layer which contains cellulose fibers, binders and superabsorber particles. Nonwoven webs comprising a combination of fluff pulp fibers, synthetic fibers, latex binder and superabsorber particles is also disclosed. As a latex binder, for example vinyl acetate-ethylene can be used.

It is the objective of the present invention to provide webs which preferably can be used as fluid acquisition layers said webs possessing the characteristics of the carded webs current available whilst offering the advantages of reduced manufacturing costs of the airlaid process.

### SUMMARY OF THE INVENTION

The present invention relates to nonwoven fabrics that can be used in the manufacture of absorbent articles and to acquisition layers useful for such articles. More specifically, the invention relates to a nonwoven fabric comprising mixed synthetic fibre and natural fibre matrices bonded by means of selected latices and laid down using the airlaid process to produce a latex bonded airlaid fabric (hereinafter "LBAL"). This is especially adapted for use as an ADL, for example in personal care products.

This invention explores the effect on performance of varying the ratio between synthetic fibres and fluff pulp consolidated with cross-linking, aqueous based polymer dispersions (homopolymers and copolymers) to produce LBAL fabric to function as an ADL.

There are many variables available for the construction of a nonwoven fabric for use as ADL. One aspect of this invention is the identification of the optimum ratios of the single components defining the nonwoven fabric, namely binder, fibre pulp and synthetic fibre to produce a high performance LBAL ADL structure.

In addition to length and thickness, synthetic fibres vary in their level of crimp, being classified as either straight - medium - high. Higher crimp allows ease of fibre separation but slows forming capacity and line speed since their elastic character allows the fibres to "bounce" inside the head assembly of an airlaying apparatus. Medium crimp types are the preferred choice for the airlaid process offering a compromise between ease of separation and line speed. A further potential variable is the ratio between long and short fibres within the overall matrix because long fibres tend to lay flat versus short fibres (e.g. 3-5 mm) which tend to orientate themselves vertically - an optimum must be sought to balance resiliency and acquisition properties.

The present invention is directed to a nonwoven airlaid fabric comprising a combination of synthetic fibres, preferably polyester fibres, and of natural fibres, preferably fluff pulp fibres and very preferred of cellulosic fluff pulp fibres, which has been consolidated by application and crosslinking of a crosslinkable polymer dispersion.

In general the synthetic fibres do not contain polymers or combinations of polymers which melt under the conditions of the airlaid process. Thus preferably the nonwovens of this invention do not contain binder fibres and/or multicomponent fibres with polymer components which melt during the web formation or during the processing after web formation.

The nonwoven airlaid fabric of this invention is a LBAL nonwoven fabric with specific physical and application properties to function as an acquisition distribution layer.

A nonwoven fabric is a manufactured sheet, web or batt of directionally or randomly orientated fibres, bonded by friction, and/or cohesion and/or adhesion, excluding paper and products which are woven, knitted, tufted, stitch-bonded incorporating binding yams or filaments, or felted by wet-milling, whether or not additionally needled.

The fibres used in the nonwoven fabrics of this invention are of man-made origin and they additionally contain a portion of natural fibres.

The nonwoven fabrics of this invention are formed by the well known airlaid process.

Commercially available fibers in general have diameters ranging from 0.0001 mm to 0.8 mm.

The nonwoven fabrics of this invention comprise man-made fibres (synthetic fibres) in combination with fibres made by nature (natural fibres).

Example natural fibres include but are not limited to: animal fibers such as wool, silk, fur, and hair; and vegetable fibers such as cellulose, cotton, flax, linen and hemp. Preferably natural fibres are cellulose fibres which are applied as fluff pulp.

The term "fluff pulp" refers to a a pulp prepared by chemical, mechanical or combined chemical and mechanical treatment, usually bleached, and known as an absorbent medium, for example in disposable diapers, bedpads and hygienic personal products.

Synthetic fibres can be derived from natural fibers or not. Example synthetic fibres, which are derived from natural fibers include but are not limited to rayon and lyocell, both of which are derived from cellulose, a natural polysaccharide fiber.

Synthetic fibres, which are not derived from natural fibres can be derived from other natural sources or from mineral sources. Examples of synthetic fibres which are derived from natural sources include but are not limited to polysaccharides, such as starch.

Examples of fibres from mineral sources include but are not limited to polyolefin fibres, such as polypropylene fibres or polyethylene fibres, to polyester fibres and to polyamide fibres, which are derived from petroleum.

The nonwoven fabrics of this invention are formed from preformed fibers, which are airlaid into webs at a distinctly subsequent point in time. The airlaying process is a drylaying process and is known.

The fibres in a nonwoven web are typically joined to one or more adjacent fibres at some of the overlapping junctions termed interstices. This includes joining fibres within each layer and joining fibres between layers when there is more than one layer. Fibres can be joined besides chemical bonding by mechanical entanglement and/odr by heat-bonding, which comprises techniques such as through-air bonding and thermobonding by use of heated calendar rolls.

Any material comprising a mixture of synthetic and natural fibres and a crosslinkable binder are suitable for providing a nonwoven fabric according to the present invention in the ratios of the components provided within the guidelines of this disclosure. The respective material must provide good recovery regarding the application and removal or external pressure.

A preferred material will have a good recovery at 20°C, a temperature which is considered representative of room temperature, and thereby representative of many transport and storage conditions. A preferred material will further have good recovery values over a broad range of temperatures, namely temperatures above 20°C. A temperature of 45°C is thought to be representative of relatively high temperature storage conditions, but the temperature is also thought to be representative for the upper end of temperatures encountered under wearing conditions. A temperature of 60°C is thought to be representative of more extreme storage and transport conditions, as encountered in particularly high climates. Acquisition layers in accordance with the present invention will not loose an substantial amount of their ability to recover from external pressure when the material is held at a temperature of 45°C to 60°C, respectively.

Preferred materials according to the present invention will have a recovery at 20°C of at least 70 %, more preferably of at least 75 % or 80 %. For preferred materials the recovery value is at 45°C will be at least 45 %, more preferably 50 %, 55 % or at least 60 %.

According to the present invention the recovery value at 45°C is at least 65 % of the recovery value at 20°C. More preferred are higher recovery values, namely of 70 %, more preferably 75 %, yet more preferably 80 % of the recovery value at 20°C.

Preferred material will also still at 60°C have recovery values which are high as compared to their recovery values at 20°C, preferably the recovery value at 60°C is at least 50 °4, more preferably 55 %, yet more preferably 60 % or 65 % of the recovery value at 20°C.

The recovery values of materials useful for absorbent articles, and in the present context of a liquid acquisition layer of such absorbent articles are relevant at different pressures. For example, a sitting baby will exert a pressure of about 2.1 kPa, however, the pressure very much depending an the baby wearing the diaper and an the circumstances of its sitting. When absorbent articles, for example diapers, are shipped in plastic packaging, they are often subjected to pressures in the range of 6 to 10 kPa, When materials useful for liquid acquisition layers are shipped in roll stock form, they maybe subjected to pressures up to 50 kPa. Without wishing to be bound by theory it is believed that the recovery behavior of a material under very high pressures is representative also for the recovery value at lower pressures. Therefore, it is believed that a material which provides excellent recovery Performance under high pressures as experienced for example by roll stock transport, will also guarantee excellent recovery behavior when taken out of a package and when being worn. Therefore, the measurements of the recovery values as described below are carried out under a pressure of 50 kPa.

The acquisition materials have been found to work best if a blend of different fibres is used. While a blend of three or more different fibres can be used, preferably a blend of two fibres is used. Nonwoven fabrics in accordance with the present invention therefore comprise a mixture of fibres of at least one type of synthetic fibres and at least one type of natural fibres in the form of a non-woven fabric which is stabilized by a selected crosslinked polymer binder. While a large variety of fibers and binders are suitable, it has been found that certain materials provide a better recovery than other materials and are therefore preferred for materials according to the present invention.

Preferred synthetic fibres according to the present invention are polyester fibers, such as polyethylene terephthalate ("PET") fibers.

Preferred natural fibres according to the present invention are cellulose fluff pulp fibres.

Such blend may comprise at least 10 to 90 weight % of the synthetic fibres and the remaining weight % portion of the natural fibres. Highly preferred according to the present invention is a blend of 40 to 60 weight % of synthetic fibres to 60 : 40 weight % of natural fibers.

The fibres used in the manfacture of the nonwoven fabrics of this invention are not stiffened by a pretreatment with a crosslinking agent. They are supplied as proprietary raw materials, i.e. the fluff pulp can be either treated or untreated in nature and the syntheteic polyester is usually supplied ready for use with a lubricating agent applied to the surface. Strength and durability in the fabric sources from the binder when cross-linked during the nonwoven manufacutring process.

Preferably, at least the synthetic fibres will exhibit a crimp, preferably a spiral-crimp. As used herein, a spiral-crimp is any three-dimenional crimp and preferably one wherein the fibres substantially assume a helical shape.

The synthetic fibres can typically vary in thickness between 3 and 30 decitex, where preferred thickness is between 4 and 12 decitex and most preferable between 6 to 8 decitex. The thickness of natural fibres is quoted as "coarseness" due to their irregular morphology, where the preferrd values are between 2 g per 10000 metres to 10 g per 10400 metres and most preferable between 3 and 5 g per 10000 metres. A value of 3.4 g per 10000 metres equates to 3.4 decitex.

The synthetic fibres and the natural fibres may be of different lengths, where preferred synthetic fibres are 4-25 mm in length, more preferably 3-12 mm in length and most preferably 5-6 mm. The natural fibres are typically shorter than the synthetic types and can range from 1.5 to 7 mm in length, more preferably 2.5 to 5 mm in length.

Preferred crimp values for synthetic fibres are between 6 tro 20 crimps per inch, more preferable 8 to 12 crimps per inch.

Without wishing to be bound by theory, it is further believed that the spiral crimping of fibres is very beneficial for their liquid acquisition and distribution behaviour. It is assumed that the spiral crimp increases the void space in an acquisition member formed by such fibres. Often, an absorbent article, when being worn, is exposed to a certain pressure exerted by the wearer, which potentially decreases the void space in the acquisition member. Having good permeability and sufficient void space available are important for good liquid distribution and transport. Also are spiral-crimped fibres believed to provide for good permeability as for a given fibre dtex value.

Nonwoven fabrics having a basis weight from 20 to 100, preferably from 30 to 80, and most preferably from 50 to 70 g/m² are preferred.

The nonwoven fabrics of the present invention are consolidated by a crosslinked latex binder. Such latexes are known in the art but have not yet used in the airlaying process together with mixtures of synthetic fibres and natural fibres to produce ADL fabrics.

The polymeric binder used in the present invention preferably is a crosslinkable emulsion polymer. By "crosslinkable" as used herein is meant a polymer that is capable of undergoing crosslinking, either by a self-crosslinking mechanism, or by the incorporation of at least one functional monomer Into the polymer backbone which can undergo a post-polymerization crosslinking reaction to increase molecular weight.

Crosslinked latex binders can also be obtained via the addition of external crosslinkers such as melamine-formaldehyde, urea-formaldehyde, phenol-formaldehyde, gloyoxal adducts, and other similar chemistries well known in the art to a polymer latex.

Preferred latexes are polymer dispersions, particularly those based on polyvinyl esters, such as polyvinyl acetate, or acrylics and vinyl acrylics.

These latexes contain either specific crosslinkable or self-crosslinking comonomers which have been incorporated into the polymer backbone during their preparation by emulsion polymerization and/or contain specific crosslinker additives which have been added subsequently to the polymer emulsion, for example in the form of crosslinker resins, in combination with crosslinking catalysts.

Polymer dispersions based on polyvinyl esters, particularly polyvinyl acetate, which are prepared by aqueous emulsion polymerization of vinyl acetate in the presence of stabilizers (protective colloids and/or emulsifiers), represent a mass product. Overviews of the preparation and application of polyvinyl acetate dispersions can be found in numerous references in the literature, such as, for example, in the Handbook of Adhesives (I. Skeist, ed.), 3rd edition, chapter 21. Also described therein is how, for example, the comonomer N-methylolacrylamide is widespread for the preparation of self-crosslinking vinyl acetate polymers.

A preferred group of emulsion polymers which are used as binders of this invention is formed by the copolymers prepared by means of free-radical emulsion polymerization, constructed predominantly on the basis of vinyl esters as principal monomer basis, and comprising comonomers containing crosslinkable N-methylol groups or their derivatives which are etherified with C₁-C₆ alkanols.

Suitable principal vinyl ester monomers include preferably vinyl formate, vinyl acetate, vinyl propionate, vinyl isobutyrate, vinyl pivalate, vinyl 2-ethylhexanoate, vinyl esters of saturated branched monocarboxylic acids having 9 to 11 carbon atoms in the acid residue, vinyl esters of relatively long-chain saturated or unsaturated fatty acids, such as vinyl laurate or vinyl stearate, for example, and vinyl esters of benzoic acid and of substituted derivatives of benzoic acid, such as vinyl p-tert-butylbenzoate. Among these monomers, however, vinyl acetate is particularly preferred.

Said vinyl esters can be used individually in the polyvinyl ester or else can be present in a mixture in a copolymer alongside one another. The fraction of the vinyl esters from this group as a proportion of the total monomer amount in the copolymer is usually at least 50% by weight, preferably at least 75% by weight.

The emulsion polymer used as a binder in accordance with the invention contains crosslinkable N-methylol groups, which are introduced by copolymerization with comonomers containing N-methylol groups. Preferred examples of such comonomers are N-methylolacrylamide. N-methylolmethacrylamide, N-methyloiallylcarbamate, N-methylolmaleimide, N-methylolmaleamic acid, and the N-methylol amides of aromatic vinyl carboxylic acids, such as N-mothylol-p-vinylbenzamide, for example. Instead of or in addition to the N-methylol group containing comonomers their derivatives which are etherified with C₁-C₆ alkanols can be used.

A preferred class of binders of the present invention are aqueous dispersion adhesives comprising copolymerised units derived from N-methylol amides of acrylic acid and methacrylic acid. The fraction of these comonomers as a proportion of the total monomer amount is up to 9.0% by weight.

Further comonomers particularly suitable for preparing the emulsion polymers used as binders in accordance with the invention are nitrogen-containing comonomers polymerizable with vinyl esters and containing N-functional groups, with the exception of the abovementioned monomers containing N-methylol groups. They include, in particular, N-ethanol(meth)acrylamide, N-propanol(meth)acrylamide, (meth)acrylamide, allylcarbamate, acrylonitrile, the N-methylol esters, N-alkyl ethers or Mannich bases of N-methylol(meth)acrylamide or N-methylolallyicarbamate, acrylamidoglycolic acid and/or its salts, methyl acrylamidomethoxyacetate, N-(2,2-dimethoxy-1-hydroxyethyl)acrylamide, N-dimethylaminopropyl(meth)acrylamide, N-methyl(meth)acrylamide, N-butyl(meth)acrylamide, N-cyclohexyl(meth)acrylamide, N-dodecyl(meth)acrylamide, N-benzyl(meth)acrylamide, p-hydroxyphenyl(meth)-acrylamide, N-(3-hydroxy-2,2-dimethylpropyl)methacrylamide, ethyl imidazolidonemethacrylate, N-vinylformamide and N-vinylpyrrolidone. The fraction of these comonomers as a proportion of the total monomer amount is, if they are present, usually up to 15°/° by weight, preferably up to 10% by weight.

Further ethylenically unsaturated comonomers particularly suitable for preparing the emulsion polymers used as binders in accordance with the invention and copolymerizable with the vinyl esters are α,β-unsaturated acids, examples being acrylic acid and methacrylic acid, and esters thereof with primary and secondary saturated monohydric alcohols having 1 to 32 carbon atoms, examples being methanol, ethanol, propanol, butanol, 2-ethylhexyl alcohol, cycloaliphatic or polycyclic alcohols and relatively long-chain fatty alcohols. It is also possible as well to use α,β-unsaturated dicarboxylic acids, examples being maleic acid, fumaric acid, itaconic acid and citraconic acid, and their monoesters or diesters with saturated monohydric aliphatic alcohols having 1 to 28 carbon atoms. The fraction of these comonomers as a proportion of the total monomer amount is, if they are present, usually up to 25% by weight, preferably up to 15% by weight.

Further comonomers particularly suitable for preparing the emulsion polymers used as binders in accordance with the invention are ethylenically unsaturated hydrocarbons, such as ethylene or α-olefins having 3-28 carbon atoms, examples being propylene and/or butylene, or vinylaromatic hydrocarbons, such as styrene, vinyltoluene and/or vinylxylene, and halogenated unsaturated aliphatic hydrocarbons, examples being vinyl chloride, vinyl fluoride, vinylidene chloride and/or vinylidene fluoride. The fraction of these comonomers as a proportion of the total monomer amount is, if they are present, usually up to 50% by weight, preferably up to 25% by weight.

Further comonomers particularly suitable for preparing the emulsion polymers used as binders in accordance with the invention are comonomers with polyethylenic unsaturation, and hence with a crosslinking action, examples being diallyl phthalate, diallyl maleate, triallyl cyanurate, tetraallyloxyethane, divinylbenzene, butane-1,4-diol dimethacrylate, triethylene glycol dimethacrylate, divinyl adipate, allyl (meth)acrylate, vinyl cratonate, methylenebisacrylamide, hexanediol diacrylate, pentaerythritol diacrylate and trimethylolpropane triacrylate or mixtures of two or more compounds from this group may be present in the polymer. The fraction of these comonomers as a proportion of the total monomer amount is, if they are present, usually up to 10% by weight, preferably up to 2% by weight.

Further comonomers particularly suitable for preparing the emulsion polymers used as binders in accordance with the invention are hydroxy-functional esters of unsaturated carboxylic acids, such as hydroxyethyl (meth)acrylate, hydroxypropyl (meth)acrylate and adducts thereof with ethylene oxide or propylene oxide. The fraction of these comonomers as a proportion of the total monomer amount is, if they are present, usually up to 25% by weight, preferably up to 15% by weight.

Further comonomers particularly suitable for preparing the emulsion polymers used as binders in accordance with the invention are comonomers which are self-crosslinking or crosslinkable by way of carbonyl groups, from the group consisting of diacetoneacrylamide, allyl acetoacetate, vinyl acetoacetate and acetoacetoxyethyl (meth)acrylate. The fraction of these comonomers as a proportion of the total monomer amount is, if they are present, usually up to 10% by weight, preferably up to 5% by weight.

A further group of suitable comonomers comprises monomers from the group of the unsaturated sulfonic acids and salts thereof, preferably vinylsulfonic acid, 2-acrylamido-2-methylpropanesulfonic acid, epoxy-functional comonomers, such as glycidyl methacrylate and glycidyl acrylate, and silicon-functional comonomers, such as acryloyloxypropyltri(alkoxy)silanes and methacryloyloxypropyltri(alkoxy) silanes, vinyltrialkoxysilanes and vinylmethyldialkoxysilanes, in which the alkoxy groups present may be, for example, ethoxy and ethoxypropylene glycol ether radicals, for example. The fraction of these comonomers as a proportion of the total monomer amount is, if they are present, usually up to 10% by weight, preferably up to 5% by weight.

The fraction of the comonomer units present in the copolymer in addition to the vinyl ester units can together amount to up to 50% by weight,

The polymer dispersions used as a binder in this invention comprise as stabilizer at least one emulsifier or a combination of at least one emulsifier and at least one protective colloid.

As an emulsifier it is possible to use nonionic and/or ionic emulsifiers in polymer emulsions employed as a binder in this invention. These emulsifiers are present typically in amounts of up to 5% by weight, based on the total amount of the monomers. Compounds suitable for this purpose can be found in relevant compilations known to the skilled worker, such as the Surfactant Applications Directory (D.R. Karsa et al., Ed., Blackie, London 1991) or in Houben-Weyl, Methoden der organischen Chemie, volume XIV/1, Makromolekulare Stoffe [Macromolecular Compounds], Georg-Thieme-Veriag, Stuttgart, 1961, pages 190 to 208.

Suitable anionic emulsifiers are, for example, alkyl sulfonates, alkylaryl sulfonates, alkyl sulfates, sulfates of hydroxylalkanols, alkyl and alkylaryl disulfonates, sulfonated fatty acids, sulfates and phosphates of polyethoxylated alkanols and alkyphenols, as well as esters of sulfosuccinic acid. Suitable cationic emulsifiers are, for example, alkyl quaternary ammonium salts, and alkyl quaternary phosphonium salts.

Examples of suitable non-ionic emulsifiers are the addition products of 5 to 50 moles of ethylene oxide adducted to stralaht-chained and branch-chained alkanols with 6 to 22 carbon atoms, or alkylphenols, to higher fatty acids, or higher fatty acid amides, or primary and secondary higher alkyl amines; as well as block copolymers of propylene oxide with ethylene oxide and mixtures thereof.

An example of a suitable protective colloid is polyvinyl alcohol, particularly polyvinyl alcohol with a degree of hydrolysis of 60-100 mol°/o, preferably from 70 to 98 mol%, and with viscosities, of the 4% strength by weight aqueous solutions at 20°C, of from 2 to 70 mPa·s.

As protective colloids it is also possible to use etherified cellulose derivatives, examples being hydroxyethylcellulose, hydroxypropylcellulose and carboxymethylcellulose.

A detailed description of further suitable protective colloids can be found in Houben-Weyl, Methoden der organischen Chemie, volume XIV/1, Makromolekulare Stoffe [Macromolecular Compounds], Georg-Thieme-Verlag, Stuttgart, 1961, pages 411 to 420. Preference is given to the predominant use of polyvinyl alcohol. Based on the total amount of the monomers, the fraction of the protective colloids is preferably from 1 % to 20% by weight, in particular from 2% to 14% by weight.

Preferred crosslinkable binders used in this invention are dispersions of copolymers of vinyl esters with crosslinkable comonomers, including N-methylolacrylamide, which crosslink with acid catalysis in combination with water-soluble strong acids or their metal salts, particularly salts of Cr(III), Al (III), Fe(III) and Zr(IV). These binders are disclosed, for example, in DE 26 20 738 A1, DE 39 42 628 A1, EP 826,008 A1 and EP 1,170,311 A1.

Another preferred group of crosslinking additives in the binders used in this invention are hydroxymethyl-substituted imidazolidinones, hydroxymethyl-substituted pyrimidinones or hydroxymethyl-substituted triazinones or their self-condensation products or mixed condensates of two or more of said compounds, or a mixture of two or more of said compounds. These systems are disclosed in WO 01/49,788 A1.

Very preferred this latter group of crosslinkers is used in aqueous dispersions based on a polymer dispersion, preferably having a pH of between 2 and 6, comprising at least one emulsion polymer containing crosslinkable N-methylol groups, derived through the incorporation of up to 9.0% by weight, based on the total monomer amount.

A very preferred class of binders used in this invention are vinyl acetate-ethylene emulsion binders comprising cross-linking groups, preferably groups derived from N-methylol containing monomers, such as n-methylol acrylamide.

Very preferably such vinyl acetate-ethylene emulsion binders have higher levels of cross-linking monomer, such as N-methylol acrylamide, and are made by a low temperature polymerization and comprise at least 6 %, and preferably at least 7 % by weight of cross-linking monomer units. These binders produce nonwoven products having high wet tensile strength, yet have low (less than 15 ppm) of formaldehyde. Binder systems of this type are disclosed in US-A-2003/0176133.

These very preferred binders are emulsion polymers comprising
a) at least 50 % by weight percent of vinyl acetate units;
b) 0 to 40 %, preferably 1 to 40 % by weight of ethylene units;
c) 6 to 20 % by weight of crosslinking monomer units;
d) 0.1 to 7 % by weight of acrylamide, methacrylamide, or a mixture thereof; and
e) 0 to 40 % by weight of other co-monomers.

Another preferred class of binders are crosslinkable polyvinylacetate homopolymers which an amount of crosslinkable groups to result in a crosslinked polymer with glass transition temperatures between 25 - 35°C.

The emulsions used as binders in this invention may be prepared using conventional batch, semi-batch or semi-continuous emulsion polymerization procedures. Batch polymerization is preferred as it generally produces higher molecular weight polymers, and higher molecular weight polymers can lead to higher strength binders.

Generally, the monomers are polymerized in an aqueous medium in the presence of the redox initiator system and at least one emulsifying agent.

If a batch process is used, the monomers are suspended in water and are thoroughly agitated while being gradually heated to polymerization temperature. The homogenization period is followed by a polymerization period during which the initiator and functional monomers including N-methylol acrylamide are added incrementally or continuously. The functional monomers are added slowly to the reaction to minimize homopolymerization of the functional monomers, and instead promote incorporation of the functional monomers into the polymer backbone. If the slow addition procedure is employed, the monomers are added gradually throughout the polymerization reaction.

The initiator system is generally a redox system, which is effective for lower temperature polymerizations. Redox systems using persulfate or peroxide initiators along with a reducing agent are preferred. Peroxide initiators, and most preferably tert-butyl hydrogen peroxide (tBHP) may be used to initiate polymerization. Preferably the redox initiator system is slow-added during the polymerization.
Instead of using redox systems thermal initiator systems can also be adopted, for example azo compounds.

To control the generation of free radicals, a transition metal often is incorporated into the redox system, and such metals include an iron salt, e.g., ferrous and ferric chloride and ferrous ammonium sulfate. The use of transition metals and levels of addition to form a redox system for polymerization mediums are well-known.

The polymerization is carried out at a pH of between 2 and 7, preferably between 3 and 5. In order to maintain the pH range, it may be useful to work in the presence of customary buffer systems, for example, in the presence of alkali metal acetates, alkali metal carbonates, alkali metal phosphates.

Polymerisation temperatures are generally within a range between 20°C and 120°C, preferably within a range between 40 to 96°C and especially preferred in the range between 50 to 90°C.

Polymerization regulators, like mercaptans, chloroform, methylene chloride and trichloroethylene, can also be added in some cases.

Useful dispersing agents are emulsifiers or a combination of emulsifiers and protective colloids generally used in emulsion polymerization.

When combinations of emulsifying agents are used, it is advantageous to use a relatively hydrophobic emulsifying agent in combination with a relatively hydrophilic agent.

The amount of emulsifying agent is generally from about 1 to 10, preferably from about 2 to about 8, weight percent of the monomers used in the polymerization.

Various protective colloids may also be used in addition to the emulsifiers described above. Examples for suitable protective colloids have been given above. In general, these colloids are used at levels of 0.05 to 4 percent by weight, based on the total emulsion.

The dispersing agent used in the polymerization may be added in its entirety to the initial charge, or a portion of the emulsifier, e.g., from 25 to 90 percent thereof, can be added continuously or intermittently during polymerization.

The polymerization reaction is generally continued until the residual monomer content is below about 1 percent, preferably less than 0.2 percent. The completed reaction product is then allowed to cool to about room temperature, while sealed from the atmosphere.

The emulsions are produced and used at relatively high solids contents, e.g., between 35 to 60 percent, preferably 50 to 55 percent, although they may be diluted with water as desired. Preferably the viscosity of the emulsion at 50 percent solids is less than 1000 cps (at 25°C).

The synthetic polymeric binders used in the present invention generally have a glass transition temperature T_{g} (measured by differential scanning calorimetry (DSC)) in the range of from -60° C to +50° C, and preferably between -40° C and +35° C.

Preferred binders show a T_{g} in the range -10°C to + 30°C, and most preferably from -0°C to + 20°C.

The film-forming polymer is present in the binder composition at from 75 to 95 percent by weight, based on the solids content of the binder composition.

The particle size of the latex can be regulated by the quantity of nonionic or anionic emulsifying agent or protective colloid employed. To obtain smaller particles sizes, greater amounts of emulsifying agents are used. As a general rule, the greater amount of the emulsifying agent employed, the smaller the average particle size.

Other adjuvants may also be present in the binder composition at a level of from 0 to 2 percent by weight. Other additives that may be incorporated into the binder composition include, but are not limited to, suspension aids, thickening agents, parting agents, penetrating agents, wetting agents, thermal gelling agents, sizing agents, defoaming agents, foam suppressors, blowing agents, coloring agents, oxidation inhibitors, quenchers, antimicrobial agents, dispersants, antistatic agents, cross linking agents (to improve wet strength), dispersants, lubricants, plasticizers, pH regulators, flow modifiers, setting promoters, and water-proofing agents, and mixtures thereof.

The emulsion binders of the invention are used to bind fibers together in a nonwoven fabric. They are used in the manufacturing process of this product which is an airlaid and chemically-bonded dry-formed web, as opposed to a mechanically tangled or thermally bonded web.

The binder is applied to the fiber by any means known in the art, such as print, foam, saturate, coating, and spraying; then dried on steam cans or ovens as currently practiced in the production of non-woven rolled goods. Binder add-on levels for non-wovens useful in the present invention can be from 5 to 40 percent, preferably from 10 to 30 percent.

Most preferred would be the spray application of the binder to the fibres in combination with drying and curing using heated ovens.

The airlaid and chemically bonded non-wovens of the present invention are useful in applications in which wet integrity or resiliency is important, such as in personal care products, for example wipes, diapers or feminine hygiene products.

Very preferably the nonwoven fabrics of this Invention are used as acquisition and distribution layers in personal care products.

These uses are also subject of the present invention.

The binder can be used as a coating or treatment on non-woven fabrics, to improve the strength and durability of the substrate, especially in contact with aqueous or non-aqueous liquids.

Without being bound by theory, it is believed that such binders are more effective because the temperature sensitivity of the recovery value for such binders is reduced compared to other binders of the prior art.

The preferred nonwoven fabrics of this invention comprise binders which are self cross-linking vinyl acetate acetate homopolymers, or self crosslinking vinyl acetate / ethylene copolymers with a glass transition temperature in the range of -15 °C to 40 °C.

Still other preferred nonwoven fabrics of this invention contain a combination of synthetic fibres and cellulose fluff pulp.

The preferred synthetic fibres are polyester types with a fibre length between 3-12 mm and 4 -12 dtex in thickness.

The polyester fibres can be straight or crimped. The cellulose used to form the fluff pulp can be treated or untreated types- The synthetic and cellulose fibres can be consolidated solely with the binder to produce a LBAL nonwoven fabric.

Preferably these components can be varied in the following dry ratios where the total adds to 100 % in any single combination:
10-40 % by weight of binder, preferably 20-30 % by weight;
10-90 % by weight fluff pulp, preferably 20-40 % by weight; and
10-70 % by weight synthetic fibres, preferably 20-40 % by weight.

The ratio of 3 - 12 mm fibres lengths can be varied in any combination between the extremes i.e. 0% 3 mm and 100% 12 mm to 100 % 3 mm to 0% 12mm.

The resulting nonwoven fabric can be used as an ADL where wet resiliency, fast liquid strike through and low re-wet characteristics are required in addition to dry and wet tensile strength plus dry compression recovery.

Preferred nonwoven fabrics show dry compression recovery ratios between 0.36 and 0.85, and wet compression recovery ratios between 0.49 and 0.63.

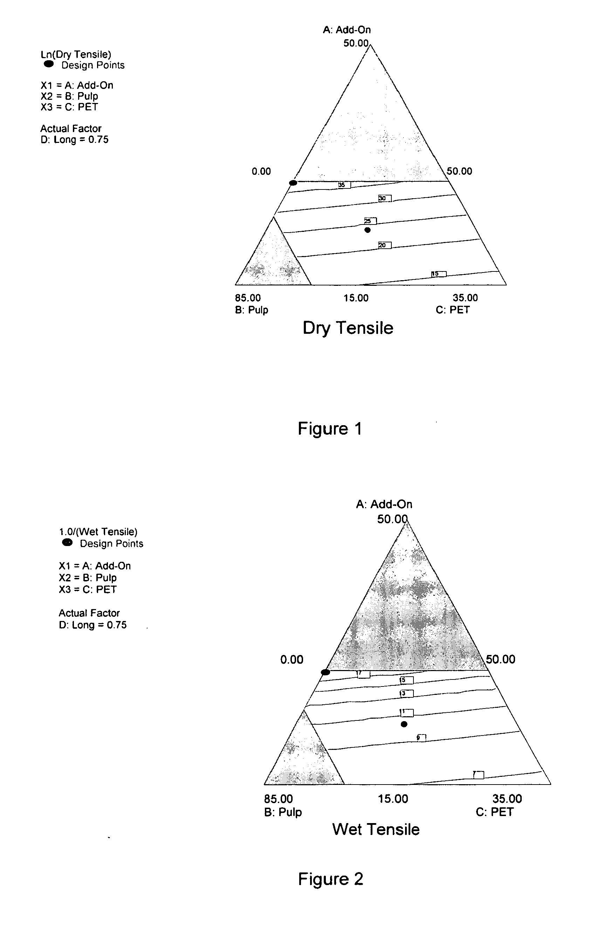

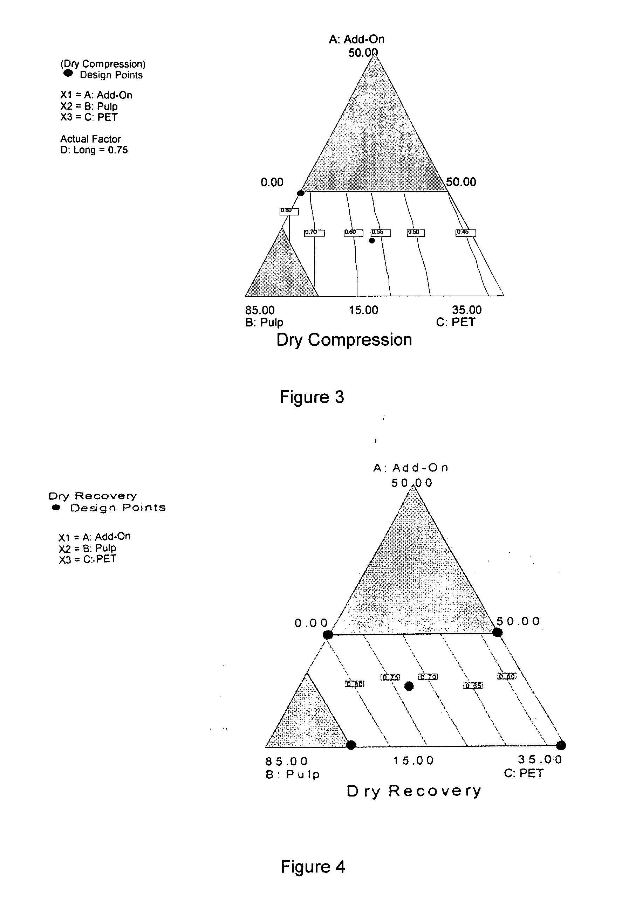

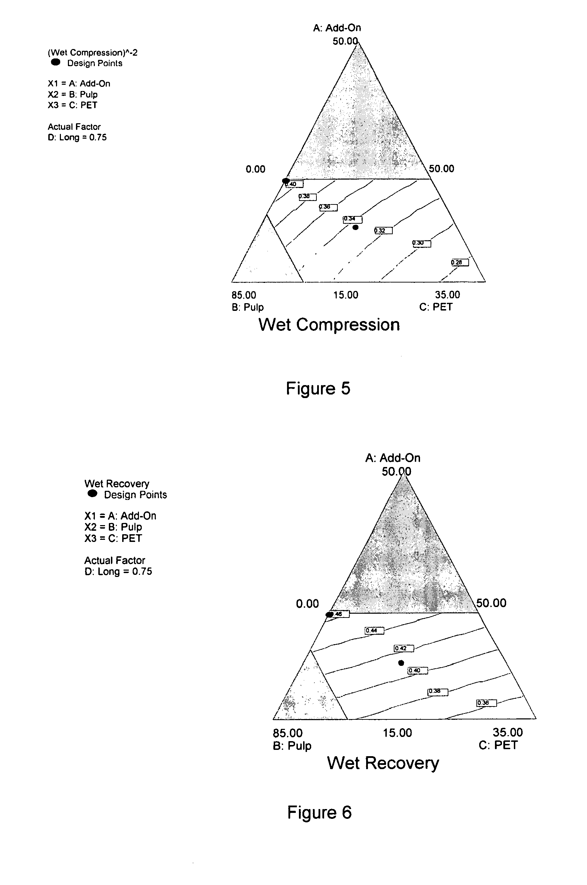

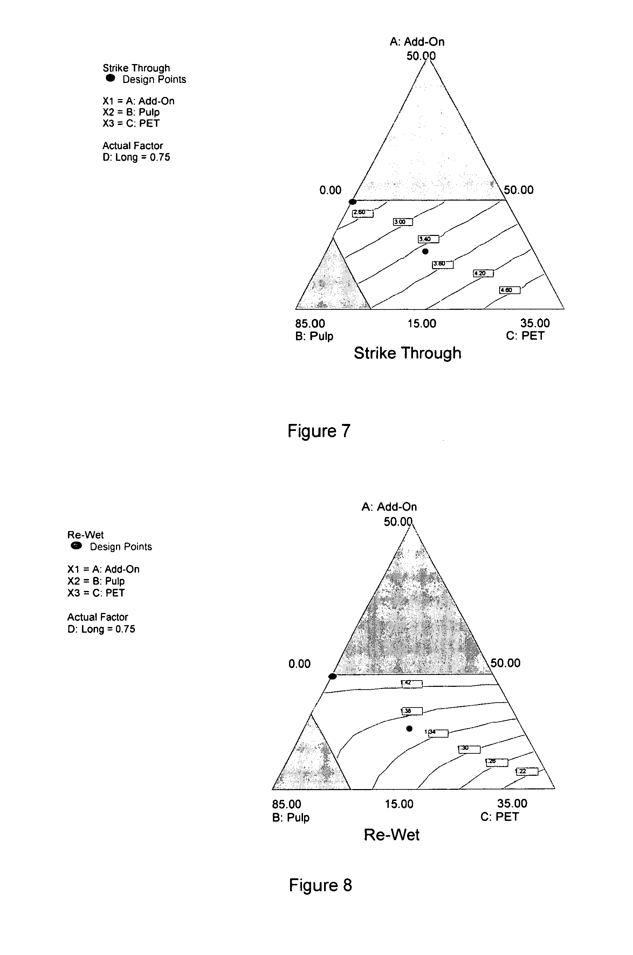

Figures 1 to 8 show response models of different properties of the nonwovens of this invention as a function of amount of binder, cellulose fluff pulp and synthetic fibre used to optimise the ratios of the components in the preferred designs.
Figure 1 shows the dependence of tensile strength from said variables.
Figure 2 shows the dependence of wet tensile strength from said variables.
Figure 3 shows the dependence of dry compression from said variables.
Figure 4 shows the dependence of dry recovery from said variables.
Figure 5 shows the dependence of wet compression from said variables.
Figure 6 shows the dependence of wet recovery from said variables.
Figure 7 shows the dependence of strike through from said variables.
Figure 8 shows the dependence of re-wet from said variables.

The data in these figures demonstrate that
- for both dry and wet strength the fibre length ratio is not significant and that the direction of isobars clearly identifies increasing strength with increasing add-on (binder content),
- for dry compression the fibre length ratio is not significant and that the direction of isobars indicate dry compression is independent of the binder content,
- for dry recovery the fibre length ratio is not significant and that the direction of isobars indicate dry recovery is influenced by the fluff pulp content,
- for wet compression the fibre length ratio is not significant and that the direction of isobars indicate reducing synthetic fibre amount and increasing binder content will boost wet compression.
- for wet recovery the fibre length ratio is not significant and that the direction of isobars indicate reducing synthetic fibre amount and increasing binder content will boost wet recovery,
- for strike through the fibre length ratio is not significant, and
- for re-wet (wet back) that the fibre length ratio is not significant and that the direction of Isobars suggests wet back is influenced by increasing binder content at the expense of synthetic fibre amount.

From these results the following conclusions can be drawn:
1. increasing the level of polyester fibre does not materially improve wet resilliency contrary to the expectations
2. The ratio of long to short poylester fibres eoes not appear to be significant, and
3. Dry and wet tensile strengths are strongly affected by increasing the amount of binder

The following examples are presented to further illustrate and explain the present invention and should not be taken as limiting in any regard. Amounts, if not indicated otherwise, are percentages by weight.

### Example 1

Different nonwoven webs were prepared by airlaid technology using as process variables amount of binder, of cellulose fluff pulp and of polyester fibres. The synthetic fibres were subdivided into long (12 mm) and short (3-5 mm) fibres.

Total number of experimental variables was 4, i.e. binder / pulp / short PET / long PET.

Several properties of the obtained nonwoven webs were investigated. These were

Compression recovery according to PE 329 test method as described below
Wet resilliency as described below
Strike through according to ERT 150.4-99 test method
Re-wet according to ERT 150.2-99 test method
ERT refers to EDANA RECOMMENDED TEST (www.edana.org)
Total number of experimental variables was 4, i.e. compression recovery / wet resiliency / strike through / re-wet.

### As a secondary test tensile strength according to ERT 20.2-89 was determined

The compression recovery according to PE 329 test method was determined as follows:
Data are based on nonwoven sample cut in pieces of 5 cm x 5 cm
   - stack samples to 3 plies
   - measure caliper at 4 corner points + centre point
   - sandwich prepared samples between release paper
   - sandwich samples prepared in release paper between wooden blocks of 10 cm x 10 cm
   - apply 1820 grams on top of sample
   - place sample in oven for 24 hrs at 60°C
   - re-measure caliper (compression)
   - allow sample to rest for 2 hours, re-measure caliper (recovery)

The wet resiliency according to modified PE 329 test method was determined as follows:

Data are based on nonwoven sample cut in pieces of 5 cm x 5 cm
- stack samples to 3 plies
- measure caliper at 4 corner points + centre point
- load fabrics with demineralised water, 10 times its dry weight
- sandwich prepared samples between release paper
- sandwich samples prepared in release paper between wooden blocks of 10 cm * 10 cm
- apply 1820 grams on top of sample
- place sample for 2 hrs at room temperature
- re-measure caliper (compression)
- allow sample to rest for 2 hours, re-measure caliper (recovery)

The design of the nonwoven webs is given in the following table 1 and the properties of said nonwoven webs are indicated in the following table 2.

**Table 1: Design of nonwoven webs**

| Example no. | binder (%)²⁾ | cellulose fluff pulp (%) | polyester fibre, short fraction (%) | polyester fibre, long fraction ¹⁾ (%) |
|---|---|---|---|---|
| 1-1 | 30 | 70 | 0 | N/a |
| 1-2 | 30 | 50 | 20 | 1 |
| 1-3 | 15 | 75 | 10 | 1 |
| 1-4 | 23 | 64 | 13 | 1 |
| 1-5 | 23 | 64 | 13 | 0.75 |
| 1-6 | 23 | 64 | 13 | 0.5 |
| 1-7 | 15 | 50 | 35 | 0.5 |

| | | | | |
|---|---|---|---|---|
| ¹⁾ 1 = all long fibre 0.75 = long:short ratio of 3:1 (3/4 long) 0.5 = long:short ratio of 1:1 (even split) ²⁾ As a binder a crosslinkable vinylacetate-ethylene copolymer was used possessing copolymerised N-methylolacrylamide units with composition in the range of VA (65-90) / E (20-30) / NMA (5-10) where the total in any combination totals 100, with mixed anionic / nonionic stabilisation where the total amount of emulsifiers is between 1.5 and 3.5 % on dry monomer weight. | | | | |

**Table 2: Characteristics of nonwoven webs**

| Example no. | Base weight (g/m²) | caliper (mm) | MD¹⁾ dry tensile strength (N/5cm) | MD wet strength (N/5cm) | dry compression | dry recovery | wet resiliency (ratios; compression) | wet resiliency (ratios; recovery) | liquid strike through & wet back |
|---|---|---|---|---|---|---|---|---|---|
| 1-1²⁾ | 56 | 1.1 | 38.5 | 19.2 | 0.74 | 0.79 | 0.42 | 0.47 | 2.03/1.44 |
| 1-2 | 54 | 0.9 | 34.1 | 17.8 | 0.45 | 0.58 | 0.34 | 0.44 | 4.02/1.45 |
| 1-3 | 56 | 1.0 | 15.8 | 7.6 | 0.69 | 0.85 | 0.34 | 0.39 | 4.21/1.37 |
| 1-4 | 53 | 1.0 | 23.9 | 10.3 | 0.57 | 0.79 | 0.32 | 0.37 | 2.06/1.34 |
| 1-5 | 55 | 1.0 | 23.1 | 9.0 | 0.57 | 0.72 | 0.33 | 0.42 | 4.72/1.29 |
| 1-6 | 53 | 0.9 | 22.9 | 10.2 | 0.55 | 0.69 | 0.33 | 0.40 | 4.32/1.42 |
| 1-7 | 53 | 1.2 | 13.4 | 6.5 | 0.44 | 0.57 | 0.27 | 0.34 | 4.48/1.18 |

| | | | | | | | | | |
|---|---|---|---|---|---|---|---|---|---|
| ¹⁾ MD denotes machine direction ²⁾ Example 1-1 is a high strength latex bonded airlaid control with no polyester fibre | | | | | | | | | |

### Example 2

In this example the level of polyester fibres was incrementally increased up to a maximum of 50 % to further investigate the previous indication that increasing synthetic fibre level does not materially enhance wet compression and recovery. It is estimated that about 50 % of polyester fibres (at 30 % binder add-on) is the maximum level that can be accommodated in the LBAL design. Above this level, in general poor transfer between the forming head and the spray boom carrier wires fractures the unbonded fibres thereby stopping the machine. At cellulose levels equal or greater than 20 %, sufficient structural integrity is retained due to the rough surface morphology compared to polyester types that are slippery and tend to separate.

In addition there was introduced a firmer hand cross-linking vinyl acetate (VA) polymer.

In addition the overall binder level was retained static at 30 % throughout the series, where the higher binder content delivered the highest strength characteristics in the preceeding series.

Furthermore there was a focus on short polyester fibres based on indications that the balance between long and short fibres does not significantly change application properties.
- allow sample to rest for 2 hours, re-measure caliper (recovery)

The design of the nonwoven webs is given in the following table 3 and the properties of said nonwoven webs are indicated in the following table 4.

**Table 3: Design of nonwoven webs**

| Example no. | binder type¹⁾ | cellulose fluff pulp (%) | polyester fibre, total amount (%) | polyester fibre, short fraction (%) |
|---|---|---|---|---|
| 2-1 | A | 50 | 20 | 100 |
| 2-2 | A | 30 | 40 | 100 |
| 2-3 | A | 20 | 50 | 100 |
| 2-4 | B | 50 | 20 | 100 |
| 2-5 | B | 30 | 40 | 100 |
| 2-6 | B | 20 | 50 | 100 |

| | | | | |
|---|---|---|---|---|
| ¹⁾ A = 25-135A which is a cross-linking VAE copolymer e.g. Celanese Emulsions Elite ULTRA B = 25-2862 which is a cross-liniking VA dispersion | | | | |

**Table 4: Characteristics of nonwoven webs**

| Example no. | Base weight (g/m²) | caliper (mm) | MD¹⁾ dry tensile strength (N/5cm) | MD wet strength (N/5cm) | dry compression | dry recovery | wet resiliency (ratios; compression) | wet resiliency (ratios; recovery) | liquid strike through & wet back |
|---|---|---|---|---|---|---|---|---|---|
| 2-1 | 56 | 1.0 | 28.4 | 14.6 | 0.38 | 0.47 | 0.39 | 0.49 | 1.39/1.65 |
| 2-2 | 54 | 1.1 | 23.0 | 11.9 | 0.39 | 0.46 | 0.43 | 0.55 | 1.26/1.71 |
| 2-3 | 58 | 1.8 | 17.1 | 8.8 | 0.33 | 0.39 | 0.45 | 0.63 | 0.96/1.62 |
| 2-4 | 54 | 1.0 | 33.5 | 15.7 | 0.44 | 0.51 | 0.38 | 0.46 | 2.85/1.70 |
| 2-5 | 56 | 1.3 | 24.5 | 13.4 | 0.37 | 0.43 | 0.36 | 0.49 | 1.63/1.72 |
| 2-6 | 55 | 1.8 | 20.1 | 9.1 | 0.31 | 0.36 | 0.35 | 0.52 | 1.25/1.68 |

in table 2 Example 2-1 represents an internal control similar to Example 1-2 of table 1 but based on solely short polyester fibres

All polyester fibres used in Examples 2-1 to 2-6 were of 6.7dtex at 6 mm length

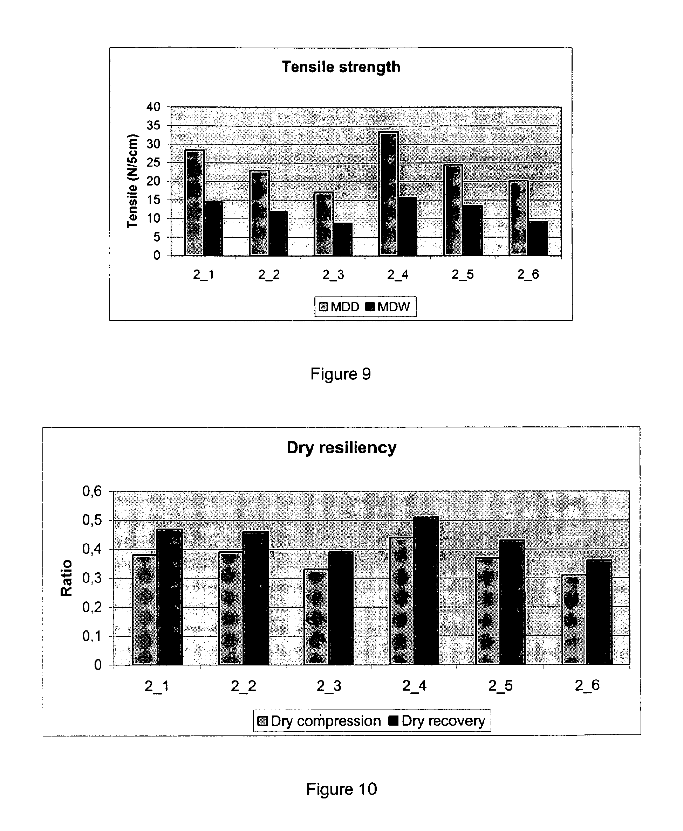

In the Figures 9 to 12 properties of the nonwovens of Examples 2-1 to 2-6 are illustrated.
Figure 9 shows tensile strength data.
Figure 10 shows dry resilency data.

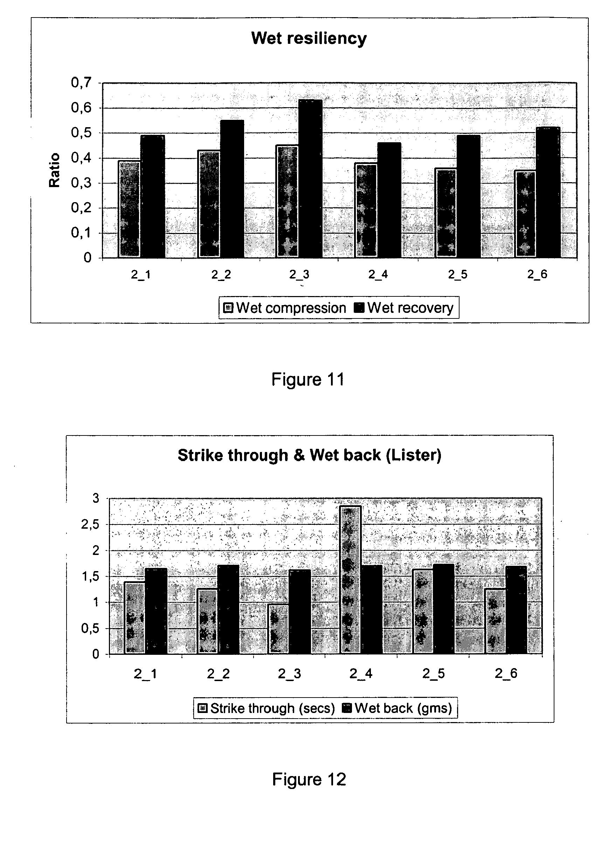

Figure 11 shows wet resilency data.
Figure 12 shows strike through and wet back data.

From the data given in Figures 9 to 12 it can be concluded that
- Using a homopolymer binder generates higher dry strength
- Increasing the level of polyester fibre affects the dry compression / recovery performance where increasing the synthetic level appears to allow greater compaction with the application of force but with a marginal reduction in the degree of recovery. This feature is most probably attributed to the higher caliper and lower density at equivalent basis weight, Inclusion of polyvinyl alcohol tends to enhance dry resiliency characteristics, and
- Wet resiliency has been improved, compared to the example no. 1 fabric designs, with increasing levels of short synthetic fibres. The inclusion of polyvinyl alcohol does not enhance wet resiliency.

Based on performance data from the combined studies, there are two fabric designs consolidated with a binder of crosslinkable vinylacetate-ethylene copolymer at high synthetic fibre loadings that exhibit very preferred properties for the target ADL sector i.e. nonwovens of Example 2-2 (30°/o binder / 40% PET / 30% pulp) and nonwovens of Example 2-3 (30% binder / 50% PET / 20% pulp).

Both offer adequate tensile properties in combination with dry resiliency plus the best wet resiliency characteristics of the combined series. Use of the selected crosslinkable homopolymer does not offer any significant advantage over crosslinkable vinylacetate-ethylene copolymer.

## Claims

1. Nonwoven airlaid fabric comprising a combination of synthetic fibres and of natural fibres which has been consolidated by application and crosslinking of a crosslinkable polymer dispersion.

2. Nonwoven fabric according to claim 1, wherein the synthetic fibres are polyester fibres, preferably polyethylene terephthalate fibres.

3. Nonwoven fabric according to claim 1, wherein the natural fibres are fluff pulp fibres.

4. Nonwoven fabric according to claim 3, wherein the fluff pulp fibres are cellulosic fluff pulp fibres.

5. Nonwoven fabric according to claim 1, wherein a blend of 40 to 60 weight % of synthetic fibres to 60 : 40 weight % of natural fibers is used.

6. Nonwoven fabric according to claim 1, wherein a the synthetic fibres exhibit a crimp, preferably a spiral-crimp.

7. Nonwoven fabric according to claim 1, wherein the crosslinkable polymer dispersion is a polymer latex based on polyvinyl esters which contain crosslinkable or self-crosslinking comonomers which have been incorporated into the polymer backbone during their preparation by emulsion polymerization and/or which contain crosslinker additives which have been added subsequently to the polymer emulsion.

8. Nonwoven fabric according to claim 1, wherein the crosslinkable polymer dispersion is formed by free-radical emulsion polymerization with vinyl esters as principal monomer basis, and the copolymers formed comprise copolymerised units containing crosslinkable N-methylol groups or their derivatives which are etherified with C₁-C₆ alkanols.

9. Nonwoven fabric according to claim 8, wherein the copolymerised units are derived from N-methylol amides of acrylic acid and methacrylic acid and the fraction of these comonomers as a proportion of the total monomer amount is up to 9.0% by weight.

10. Nonwoven fabric according to claim 1, wherein the crosslinkable polymer dispersion comprises as a stabilizer at least one emulsifier, preferably an anionic emulsifier and/or a nonionic emulsifier.

11. Nonwoven fabric according to claim 10, wherein the crosslinkable polymer is a copolymer of vinyl esters with crosslinkable comonomers, including N-methylolacrylamide, which crosslink with acid catalysis in combination with water-soluble strong acids or their metal salts, particularly salts of Cr(III), Al(III), Fe(III) and Zr(IV).

12. Nonwoven fabric according to claim 10, wherein the crosslinkable polymer dispersion is a vinyl acetate-ethylene emulsion binder comprising cross-linking groups, preferably groups derived from N-methylol containing monomers.

13. Nonwoven fabric according to claim 10, wherein the crosslinkable polymer dispersion is a vinyl acetate-ethylene emulsion binder comprising cross-linking groups, preferably groups derived from N-methylol containing monomers, very preferred derived from N-methylol acrylamide.

14. Nonwoven fabric according to claim 12, wherein the vinyl acetate-ethylene emulsion binder has a level of cross-linking monomer of at least 6 %, and preferably at least 7 % by weight of cross-linking monomer units.

15. Nonwoven fabric according to claim 14, wherein the vinyl acatate-ethylene emulsion binder comprises
a) at least 50 % by weight percent of vinyl acetate units;
b) 1 to 40 % by weight of ethylene units;
c) 6 to 20 % by weight of crosslinking monomer units;
d) 0.1 to 7 % by weight of acrylamide, methacrylamide, or a mixture thereof; and
e) 0 to 40 % by weight of other co-monomers.

16. Nonwoven fabric according to claim 1, wherein the crosslinked polymer dispersion has a glass transition temperature in the range from -10°C to + 30°C, and most preferably from -0°C to + 20°C.

17. Nonwoven fabric according to claim 1, wherein the crosslinkable polymer dispersion is a cross-linking vinyl acetate acetate homopolymer, or a self-crosslinking vinyl acetate / ethylene copolymer with a glass transition temperature in the range of -10 °C to +30 °C.

18. Nonwoven fabric according to claim 1, wherein the synthetic fibres are polyester types with a fibre length between 3-12 mm and 4-12 dtex in thickness.

19. Use of a -nonwoven fabric according to claim 1 as a personal care product.

20. Use according to claim 19, wherein the personal care product is a diaper or a feminine hygiene product.

21. Use according to claim 19, wherein the nonwoven fabric is used as an acquisition and distribution layer in personal care products.
